# EUROPEAN PATENT APPLICATION

(11) **EP 2 468 846 A1**
(43) Date of publication of application: **27.06.2012**
(21) Application number: 10196532.5
(22) Date of filing: 22.12.2010
(51) Int. Cl.: C12M 3/00, A61F 2/24, C12N 5/00

(54) **A method of examining tissue growth and conditioning of cells on a scaffold and a perfusion bioreactor**

(71) Applicant: Ludwig-Maximilians-Universität München, 80539 München (DE)
(72) Inventor: Akra, Bassil, 81249, München (DE); Haas, Ulrike, 82166, Gräfelfing (DE); Hollweck, Trixi, 80809, München (DE)
(74) Representative: Lucke, Andreas

(57) **Abstract**

Disclosed herein is a method of examining tissue growth and/or conditioning and/or adhesion properties of cells on a synthetic and/or natural scaffold (30) as well as a perfusion bioreactor (10) for use in this method. The method comprises the following steps: fixing a scaffold (30) to a holding means (28) disposed or disposable in a perfusion bioreactor (10), generating a flow of nutrition solution along the surface of said scaffold (30) held by said holding means (28), generating and/or maintaining physiological conditions in said nutrition solution flowing along said scaffold (30) using heat exchange means (20, 24, 48, 50) and/or gas exchange means (52, 54, 56, 58, 60, 62, 64) associated with said bioreactor (10), such as to allow growth and/or conditioning of cells on said scaffold (30), and optically inspecting, in particular by microscopy, the scaffold (30) held by said holding means (28) through a window (66, 68, 70, 72) provided in said perfusion bioreactor (10) at different times or continuously during tissue growth and/or conditioning of cells on said scaffold (30) while maintaining said physiological conditions in said perfusion bioreactor (10).

## Description

### FIELD OF THE INVENTION

The present invention relates to the field of tissue engineering. In particular, the invention relates to a method of examining tissue growth and/or conditioning and/or adhesion properties of cells on a scaffold, and a perfusion bioreactor to be employed in such method. Herein, the scaffold may be either one of a synthetic or a natural scaffold.

### RELATED PRIOR ART

Worldwide, every year approximately 300.000 heart valves are implanted to patients. Currently, there are three different types of implants available, namely mechanical heart valves, biological heart valves and homografts. While mechanical artificial heart valves have good lifetimes, unfortunately, they bear a severe risk of thrombosis, meaning that patients with artificial mechanical heart valves have to use anticoagulation medication throughout their lives. This is highly undesirable, because due to the decreased coagulation, any injury or surgery involves a significant risk or danger for the patient.

While biological heart valves and homografts have good haemodynamic properties, unfortunately their lifetime is rather limited. In addition, there is a risk that the patient's body does not accept the biological homograft implant or that an inflammatoric reaction may occur.

In order to overcome the abovementioned difficulties, it has been attempted to produce artificial heart valves via tissue engineering. Tissue engineering utilizes living cells as engineering materials. For example, if an artificial heart valve is to be produced by tissue engineering, an artificial heart valve is first seeded with smooth muscle cells, then with fibroblasts and finally with endothelium cells, corresponding to the structure of human vessels. It has been shown that with this three layer construction, the attachment of the endothelium cells has been improved as compared to a simple construction, but the attachment is unfortunately generally not strong enough to withstand shear forces that will occur during operation (see Fang Ning-tao, Xie Shang-zhe, Construction of tissue-engineered heart valves by using decellularized scaffolds and endothelial progenitor cells, Chin Med. 120, 2007, Vol. 8, pp. 696-702). It has been observed that the formation of the extra cellular matrix can be improved if the artificial heart valve is subjected to mechanical strain or a constant fluid flow (see Ralf Sodian, Thees Lemke, Matthias Loebe, Simon P. Hoerstrup, New Pulsatile Bioreactor for Fabrication of Tissue-Engineered Patches, Tissue Engineering, March 9, 2001, pp. 401-405), thereby allowing for an increased stability of the cell layers. This effect has been employed in a certain class of bioreactors, in which the implant is subjected to fluid flow and mechanical strain. Under these conditions, the endothelium cells will align according to the flow direction of the fluid flow (see Helmut Gulbins, et al, Development of an artificial vessel lined with human vascular cells, The Journal of Thoracic and Cardiovascular Surgery, September 2004, pp. 372-377).

Bioreactors for use in tissue engineering are for example disclosed in DE 199 19 625 C2, DE 103 49 688 A1, DE 103 22 024 A1 and DE 100 53 014 A1.

While remarkable success has been made employing such prior art bioreactors, tissue engineering is still a fairly new discipline which is based on the principle of trial and error. In particular, still much research has to be done to find out optimum combinations of scaffolds, cell types and growth factors. For example, it may very well be that certain cells grow well on certain scaffolds under statical conditions, but will not withstand the physiological flow occurring when the tissue is employed in the vascular system.

Unfortunately, determining the best combinations of scaffold, cells and tissue growth conditions is a very time consuming task. For example, seeding an artificial heart valve takes about 2.000.000 cells/cm² that have to be cultivated in advance. Whether the tissue will eventually be capable of withstanding the fluid-dynamical stress in the physiological environment can typically only be determined from the confluently colonized valve scaffold. This means that it may well take one month to grow a tissue that then turns out to be entirely useless. These problems are not limited to tissue engineering for heart valves or cardiovascular applications in general, which have been referred to above merely by way of example, but to practically all types of tissue engineering.

### SUMMARY OF THE INVENTION

It is hence an object of the invention to provide a method and an apparatus for examining tissue growth and/or conditioning and/or adhesion properties of cells to determine promising combinations of scaffold and cells with less experimental effort. In the following, whenever reference to the examination of conditioning of cells is made, this is meant to also refer to the examination of adhesion properties of the cells or detachment of the cells, without further mention.

This object is achieved by a method according to claim 1 and a perfusion bioreactor of claim 4. Preferable embodiments are defined in the depending claims.

The method of the invention comprises the following steps:
- fixing a scaffold to a holding means disposed or disposable in a perfusion bioreactor,
- generating a flow of nutrition solution along the surface of said scaffold held by said holding means,
- generating and/or maintaining physiological conditions in said nutrition solution flowing along said scaffold using heat exchange means and/or gas exchange means associated with said bioreactor, such as to allow tissue growth and/or conditioning of cells on said scaffold, and
- optically inspecting, in particular by microscopy, the colonized scaffold held by said holding means through a window provided in said perfusion bioreactor at different times or continuously during tissue growth and/or conditioning of cells on said scaffold while maintaining said physiological conditions in said perfusion bioreactor.

According to the invention, the success and/or stability of tissue growth or conditioning can be optically inspected during the tissue growth and/or conditioning process itself, i.e. the scientist does not have to wait with evaluations until the growth of tissue and/or conditioning is finished. This is enabled by a new type of bioreactor that allows to simultaneously optically inspect the tissue through a window and at the same time maintain physiological conditions inside the bioreactor, so that tissue growth and/or conditioning may continue during or after the inspection. Accordingly, less promising combinations of scaffolds and cells can be recognized early on, and the experiment can be terminated at an early stage, without spending too much effort in vain. Herein, the "scaffold" may be a sample piece of a "full" or "true" scaffold for actual use in tissue engineering, and in particular, a sample piece of a scaffold for cardiovascular applications, such as a (possibly comparatively small) sample piece of a heart valve or a cardiovascular patch scaffold. However, the method is not limited to this and may be applied for scaffolds for generally any type of tissue.

Also, by generating a flow of nutrition solution along the surface of said colonized scaffold held by said holding means, the effect the flow of nutrition solution has on the tissue can be directly observed. For example, by varying the flow during the experiment, the effect of the flow on the tissue growth and/or the conditioning can be directly examined. Also, by adjusting the flow of nutrition solution to flow rates similar to the physiological environment of the intended use, it can be directly determined whether the tissue will withstand the fluid dynamical stress it will encounter after implantation.

In summary, by allowing for optical inspection of the tissue at any time during tissue growth and/or conditioning, valuable additional information can be obtained, information can be obtained much earlier, and a better understanding of the tissue growth and/or conditioning process and material biocompatibility properties can be gained. Accordingly, more empirical information can be obtained with less experimental effort.

In a preferred embodiment, the flow of nutrition solution along said scaffold is adjusted to a flow speed of 0.04 to 167 mm/s, preferably at least 33 mm/s. These are flow speeds which may realistically be expected in-vivo e.g. in the cardiovascular system. The method and apparatus of the invention allow to directly verify whether the tissue would withstand the shear forces generated by such flow. However, it is to be understood that the method is not limited to tissue engineering for cardiovascular applications.

In a preferred embodiment, the scaffold used in the method is comparatively small and has a size of 400 mm² or less, preferably 200 mm² or less. For such small sample scaffolds, much less cells have to be cultivated as compared to the actual tissue to be grown. However, it turns out that such small samples are sufficient to get a very realistic and reliable impression of the behavior of the tissue, in particular in view of the possibility of direct optical inspection and control of the nutrition solution flow rate. Accordingly, a large number of combinations of scaffolds and cells can be examined in experiment with moderate experimental effort.

The invention also relates to a perfusion bioreactor for use in the above method. The perfusion bioreactor comprises a housing, holding means disposed or disposable in said housing for holding a scaffold to be seeded with cells, means for directing a flow of nutrition solution along the surface of a scaffold when held in said holding means, and at least one window, preferably two opposite windows, said at least one window being transmissive for at least one of visible light, IR or UV radiation and arranged such as to allow for optical, particularly microscopy inspection of the scaffold when held in said holding means.

Preferably, the bioreactor further comprises heat exchange means and/or gas exchange means for use in generating or maintaining physiological conditions in the nutrition solution flowing along said scaffold.

Preferably, the housing has an opening for inserting the holding means to and removing said holding means from the housing, and a lid or cap for closing the opening. Accordingly, the scaffold patch can be easily fixed in the holding means while the holding means are outside the housing, and then the holding means can be inserted to the housing with the scaffold patch fixed therein or thereon.

Preferably, the holding means comprises clamping means for conveniently fixing the scaffold by clamping. The clamping means may for example comprise an annular seat for receiving said scaffold and an annular clamping ring that can be clamped in a position suitable for pressing said scaffold against said seat. The clamping ring may further define the area of the scaffold to be colonized or seeded by cells. The clamping ring may in particular be dimensioned to restrict the scaffold area to be seeded to 400 mm² or less, preferably 200 mm² or less, and most preferably 100 mm² or less.

In a preferred embodiment, the holding means is part of a preferably cylindrical substrate chamber through which said nutrition solution may be fed and that is closed by said at least one window. The holding means may further comprise a channel for directing nutrition solution to flow along the surface of the scaffold. In this embodiment, the holding means thus forms a separate chamber, in which the desired flow conditions of the nutrition solution can be reliably and reproducibly generated. By providing the channel in the holding means as a sole entry to the substrate chamber, the flow rate of the nutrition solution into the substrate chamber can be controlled. Since the geometry of the substrate chamber is known, the flow rate of nutrition solution through the channel into the substrate chamber translates directly into a flow velocity along the scaffold. Accordingly, a predetermined flow velocity of the nutrition solution along the scaffold can be generated by controlling the flow rate of nutrition solution through said channel. This in turn allows to conduct experiments with precisely defined and reproducible flow velocities along the scaffold.

Preferably, the perfusion bioreactor further comprises inlet and outlet ports for nutrition solution, a first channel for guiding nutrition solution from said inlet port to the holding means and/or a second channel for guiding nutrition solution from said holding means to said outlet port.

Further, the heat exchange means preferably comprise inlet and outlet ports for feeding and discharging a heating fluid to and from said bioreactor, respectively. Herein, the heating fluid inlet port is preferably located closer to the nutrition solution outlet port, and the heating fluid outlet port is located closer to the nutrition solution inlet port. This way, the flow directions of the nutrition solution and the heating fluid are generally opposite to each other, hence allowing for an increased efficiency of the heat exchange.

Note that with such ports, the perfusion bioreactor can be easily combined with external supplies for nutrition solution and heating fluid, respectively. Accordingly, the experimental setup can be easily assembled, and the bioreactor can be quickly exchanged, thereby allowing scientists to carry out different experiments with different cells and scaffolds quickly one after another by simply exchanging the perfusion bioreactors. In fact, as will be shown below with regard to a specific embodiment, the perfusion bioreactor of the invention can be manufactured at very reasonable costs so that in practice it may be provided as a disposable article. By providing the ports at the perfusion bioreactor, such one-way product can be easily combined with stationary equipment for providing the nutrition solution and/or the heating fluid.

In a preferred embodiment, the housing of the perfusion bioreactor comprises a cavity for receiving said heating fluid, and at least a part of said first and/or second channel(s) is (are) disposed in said cavity such as to be at least partially surrounded by said heating fluid. This way, the temperature of the nutrition solution can be easily and efficiently adjusted upon flow through the first and/or second channels within the bioreactor.

Preferably, the heating fluid receiving cavity is partitioned into at least two compartments by at least one partitioning wall, wherein said partitioning wall has at least one opening allowing a flow of heating fluid from one compartment to another. By providing separate compartments with restricted flow from one compartment to another, a more uniform temperature distribution in the heating fluid inside the bioreactor is achieved.

Preferably, the first and/or second nutrition solution channel(s) is (are) formed by a bore in said partitioning wall. This is a very cost-efficient way of providing the channel within the bioreactor and at the same time ensures an efficient heat exchange between the heating fluid and the nutrition solution guided in said first and/or second channel(s).

In a preferred embodiment, the gas exchange means comprises one or more gas exchange interfaces for exchanging gas with said nutrition solution. Herein, the one or more gas exchange interface(s) is (are) preferably associated with the first and/or the second nutrition channels, thereby allowing gas exchange with the nutrition solution while guided through said first and/or second channels.

The one or more gas exchange interface(s) preferably comprise(s) a gas-permeable membrane, and is (are) connected with a gas inlet or outlet port via a gas channel. Accordingly, the gas exchange can be easily integrated within the perfusion bioreactor and only needs to be connected with a gas supply via the gas inlet or outlet ports.

Preferably, the gas channel is formed by a bore in a housing wall. Herein, the term "housing wall" may refer to any bottom, top or side wall or lid of the perfusion bioreactor housing. Again, using a bore in a housing wall for the gas channel is favorable from a manufacturing point of view, as it allows to reduce the number of parts, the manufacturing and the assembly costs.

Preferably, the length and width of the perfusion bioreactor housing corresponds to the length and width of a standard microtiter plate, also referred to "microplate" in the art. Microtiter plates or microplates are flat plates with multiple wells used as small test tubes. Microplates have become a standard tool in analytical research and clinical diagnostic testing laboratories, and typically have 6, 12, 24, 96 or 384 sample wells arranged in a 2:3 rectangular matrix. Since the microplates have become a de facto standard, most microscopes will be equipped to handle the same. For example, most microscopes have means for receiving and holding microplates during microscopy. By adapting the size of the bioreactor to this format, the bioreactor of the invention can be most easily examined with a conventional microscope that need not be further adapted in any particular way.

Importantly, the bioreactor as a whole can be easily placed on a microscope table to inspect the cell growth and/or conditioning on the scaffold, without interrupting the tissue growth and/or conditioning, since the bioreactor comprises heat and/or gas exchange means that allow to maintain the physiological conditions even if the bioreactor is transferred to a microscope. Accordingly, the bioreactor of the invention allows to optically inspect the scaffold held in said holding means at different times or continuously during tissue growth and/or conditioning while maintaining physiological conditions in the perfusion bioreactor. Accordingly, valuable information can be obtained not only after the tissue growth and/or conditioning is finished, but at any time during tissue growth and/or conditioning. This way, additional process related information can be obtained, which will promote a better understanding the processes of tissue growth and/or conditioning and will help to adapt the environment, particularly the flow velocity of the nutrition solution such as to obtain an optimal tissue growth and/or conditioning. Last but not least, visual inspection during an early stage of tissue growth and/or conditioning will allow to terminate an experiment early, if it is apparent that the tissue growth and/or conditioning is not satisfactory, which allows to save time and resources.

### BRIEF DESCRIPTION OF THE FIGURES

- Fig. 1: is a perspective, partly transparent view of a perfusion bioreactor according to an embodiment of the invention,
- Fig. 2: is a perspective exploded view of the components of the bioreactor of Fig. 1, seen at an angle from above,
- Fig. 3: is a perspective exploded view of the components of the bioreactor of Fig. 1, seen at an angle from below,
- Fig. 4: is a plan view onto a bottom portion of the bioreactor housing, and
- Fig. 5: is a perspective view of holding means for holding a scaffold.

### DESCRIPTION OF THE PREFERRED EMBODIMENT

For the purposes of promoting an understanding of the principles of the invention, reference will now be made to the preferred embodiment illustrated in the drawings, and specific language will be used to describe the same. It will nevertheless be understood that no limitation of the scope of the invention is thereby intended, such alterations and further modifications in the illustrated device and method and such further applications of the principles of the invention as illustrated therein being contemplated therein as would normally occur now or in the future to one skilled in the art to which the invention relates.

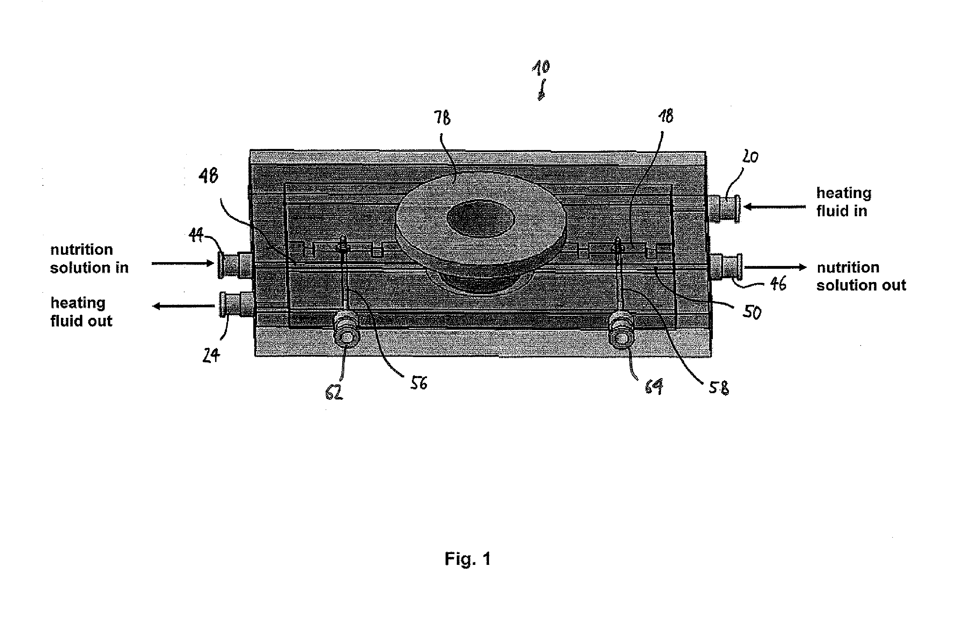

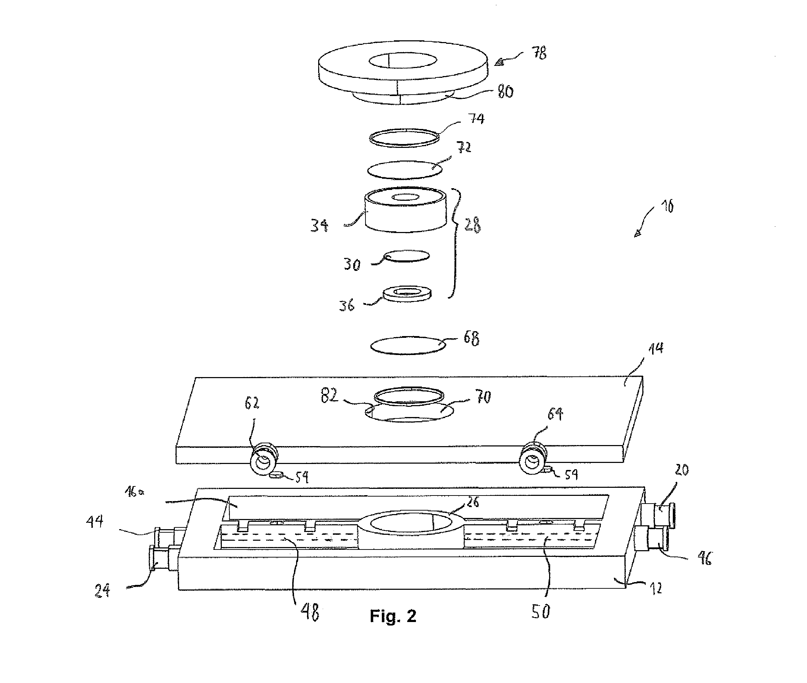

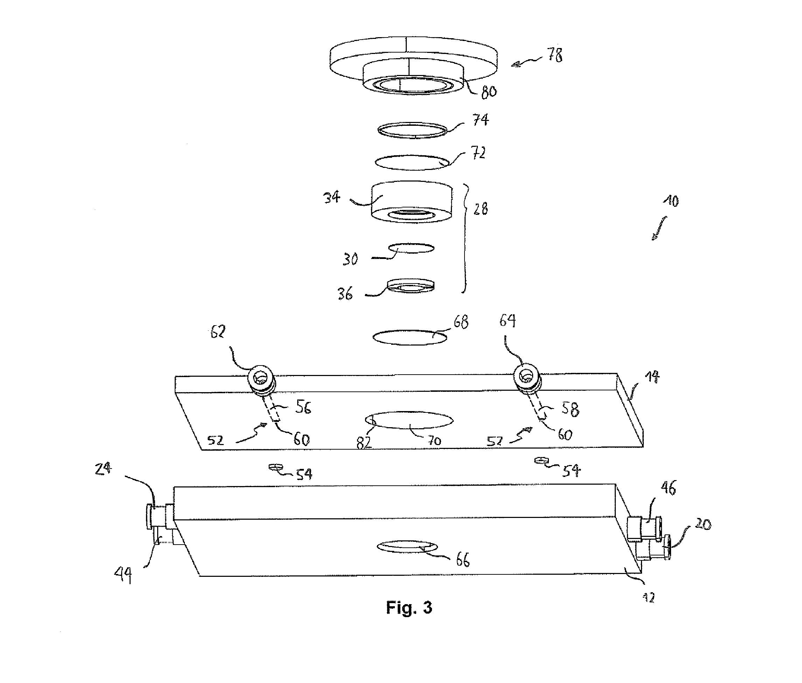

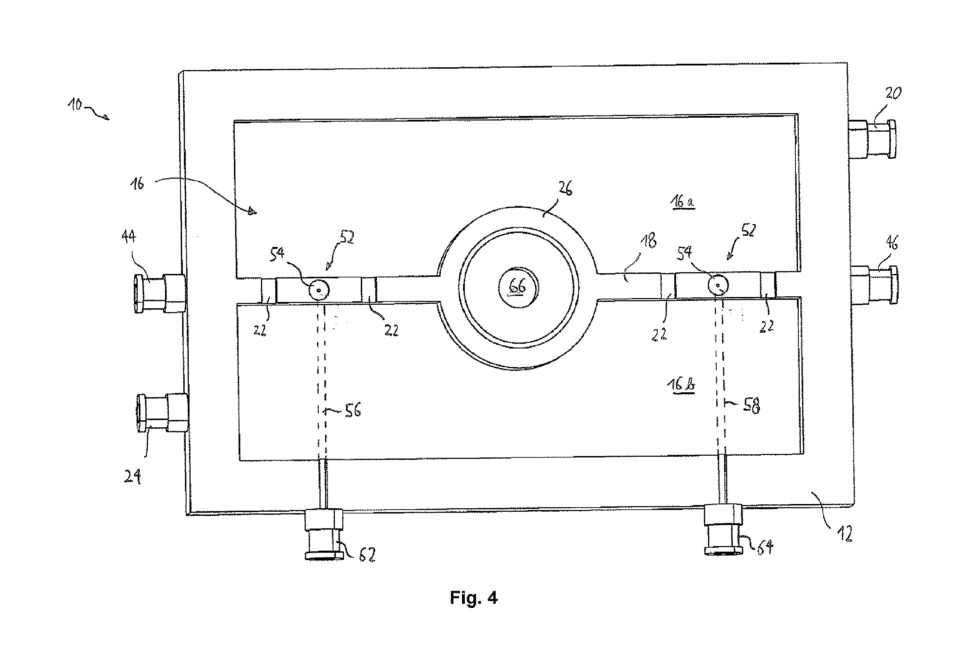

Fig. 1 shows a perspective and partially transparent view of a bioreactor 10 in the assembled state according to an embodiment of the invention. The same bioreactor 10 is also shown in Fig. 2 which is a perspective, exploded view of the bioreactor 10 as seen from an upper angle, and in Fig. 3, which is the same exploded view as Fig. 2 but seen from a lower angle. Fig. 4 is a plan view onto a bottom portion of the bioreactor 10 of Figs. 1 to 3.

As is best seen Figs. 2 and 3, the bioreactor 10 comprises a bottom portion 12 and a top portion 14. The bottom portion 12 forms a cavity 16 for receiving a heating fluid (not shown) that is divided into a first compartment 16a and a second compartment 16b by a partitioning wall 18. A heating fluid inlet port 20 with a Luer lock mechanism is provided for connecting the bioreactor 10 to a heating fluid supply (not shown), such as a supply of water at 37°C. From the inlet port 20, the heating fluid flows to the first compartment 16a, from there through narrow openings 22 in the partitioning wall 18 to the second compartment 16b and is discharged from the second compartment 16b through a heating fluid outlet port 24.

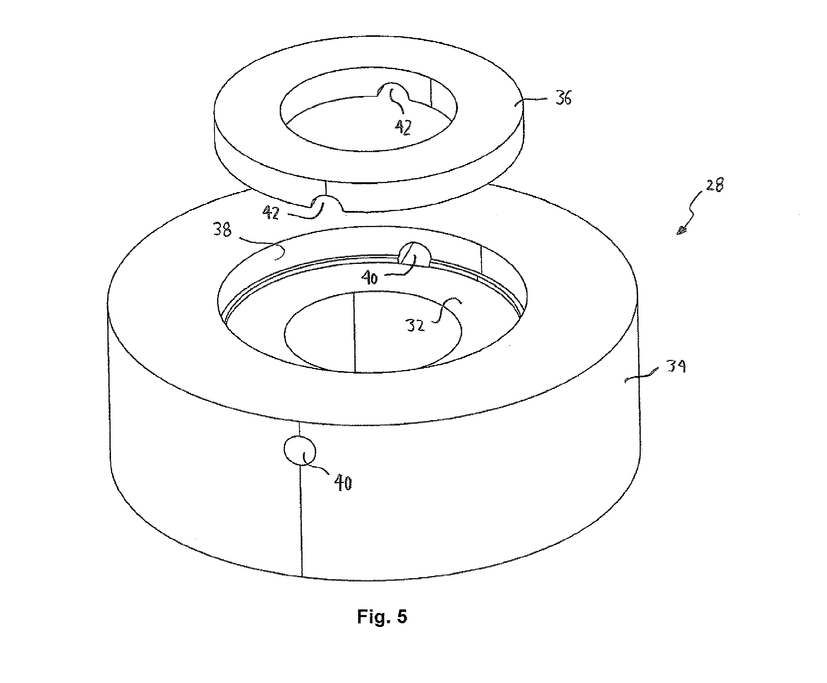

In the center of the bottom portion, a cylindrical structure 26 is formed. The cylindrical structure 26 is for receiving the holding means 28 for holding a small scaffold patch 30 (s. Figs. 2 and 3), said scaffold patch 30 typically having a diameter of only little more than 8 mm. In Fig. 5, the holding means 28 is separately shown in a perspective view. Note that the orientation of the holding means as depicted in Fig. 5 is upside down as compared to the orientation in Figs. 2 and 3.

With reference to Fig. 5, the holding means 28 comprise clamping means for clamping the scaffold 30 (s. Figs. 2 and 3, not shown in Fig. 5), which clamping means comprise an annular seat 32 in a cylindrical body 34 and a clamping ring 36. For fixing the scaffold patch 30 in the holding means 28, the scaffold patch 30 is placed on the annular seat 32 and the clamping ring 36 is pressed into an enlarged diameter portion 38 adjacent to the annular seat 32 and clamped in position, thereby pressing the scaffold patch 30 against the annular seat 32 and fixing it in place. The clamping ring 36 further defines the area of the scaffold 30 that may be seeded or colonized by cells to a desired value, such as 400 mm² or less, preferably 200 mm² or less, and most preferably 100 mm² or less.

As is further seen in Fig. 5, the cylindrical body 34 comprises a channel 40 for guiding nutrition solution to the inside of the cylindrical body 34, said channel 40 being positioned such as to direct a flow of nutrition solution along the surface of the scaffold patch 30 fixed in the holding means 28. Note that the clamping ring 36 has corresponding recesses 42 such as not to occlude the channel 40 in the cylindrical body 34.

With reference again to Figs. 1 to 4, the bioreactor 10 comprises inlet and outlet ports 44, 46 for nutrition solution. The inlet/outlet ports 44, 46 are in fluid communication with first and second channels 48, 50, respectively, that are formed by bores in the partitioning wall 18 and shown by broken lines in Fig. 2. The first and second channels 48, 50 are also in fluid communication with the channel 40 in the cylindrical body 34 of the holding means 28. The nutrition solution inlet and outlet ports 44, 46 both have a Luer lock mechanism for easy and standardized connection to an external nutrition solution supply.

Associated with the first and second channels 48, 50, are respective gas exchange interfaces 52. The gas exchange interfaces 52 comprise a water-tight but gas-permeable membrane 54 that can for example be made from silicone. The gas-permeable membrane 54 separates the first and second nutrition solution channels 48, 50 from the first and second gas channels 56, 58 formed as bores in the top portion 14. The first and second gas channels 56, 58 are shown in Fig. 1 and also shown in broken lines in Fig. 3 and Fig. 4. The outlets 60 of the first and second gas channels 56, 58 at the lower surface of the top portion 14 are seen in Fig. 3. The first and second gas channels 56, 58 are connected with respective gas inlet and gas outlet ports 62, 64 disposed at the top portion 14.

As is seen in Figs. 3 and 4, an opening 66 is formed in the bottom portion 12 and located in the center of the cylindrical structure 26. The opening 66 is hermetically sealed by a light transparent glass plate 68 and sealing means (not shown). The glass plate 68 forms a window allowing optical inspection of the scaffold 30 held in said holding means 28.

Likewise, an opening 70 is formed in the top portion 14 and is closed by a light transparent glass plate 72, a sealing ring 74 and a lid 78 that has a threaded portion 80 to be screwed into a corresponding threaded portion 82 of the opening 70. The opening 70 and the glass plate 72 form a second window for optical inspection. For example, the scaffold patch may be illuminated through the opening 70 in the top portion 14, and observed using a microscope through the opening 66 in the bottom portion 12. The opening 70 is dimensioned such that the holding means 28 can be inserted therethrough and placed in the cylindrical structure 26, with the channels 40 communicating with the first and second channels 48, 50 provided in the partitioning wall 18. In the present disclosure, the scaffold 30 may be either one of a natural or a synthetic scaffold or a combination thereof. Next, the glass plate 72 and the sealing ring 74 can be placed on top of the holding means 28 and the lid 78 may be screwed into the opening 70, thereby sealing the bioreactor 10.

Next, a method of examining tissue growth and/or conditioning of seeded cells and/or adhesion properties on the scaffold 30 will be described. Positioning of the holding means 28 for alignment of its channels 40 with the first and second channels 48, 50 may be facilitated by a notch-and-feather mechanism or the like (not shown). First, the holding means 28 is taken out of the housing, and a scaffold 30 to be seeded with cells is placed on the annular seat 32 of the cylindrical body 34 of the holding means 28 and clamped in place by inserting the clamping ring 36 to the enlarged diameter portion 38. The lid 78 is screwed from the top portion 14 and the holding means 28 is inserted through the opening 70 into the cylindrical structure 26 in a way such that the channels 40 of the holding means 28 are aligned with the first and second nutrition solution channels 48, 50. The glass plate 72 and the sealing ring 74 are placed on top of the holding means 28 and the lid 78 is closed by screwing its threaded portion 80 into the threaded portion 82 of the opening 70. The heating fluid ports 20 and 24 are connected to corresponding heating fluid supply lines (not shown) of a heating fluid circle. Typically, water at a temperature of about 37°C will be used as heating fluid.

Note that the water entering the heating fluid inlet port 20 will first enter the first compartment 16a. In order to flow into the second compartment 16b, the water must pass the narrow openings 22. This will cause an improved mixing of water already present in the first compartment 16a with fresh water supplied through the inlet port 20.

Further, supply lines (not shown) for nutrition solution are connected with the nutrition solution inlet and outlet ports 44, 46. Nutrition solution will flow through the first channel 48 formed inside the partitioning wall 18 and will hence exchange heat with the water in the first and second compartments 16a, 16b such as to ensure a physiological temperature of the nutrition solution. The nutrition solution leaving the first channel 48 passes through the channel 40 in the cylindrical body 34 of the holding means 28 and is guided by said channel 40 to flow along the surface of the scaffold 30 fixed in the holding means 28. Since the flow of nutrition solution is confined to a volume defined by the cylindrical body 34 also referred to as "substrate chamber" or "sample chamber", herein, a precisely controllable flow of nutrition solution over the surface of the scaffold patch 30 can be generated. The flow velocity can be easily adjusted by the flow rate of nutrition solution entering the inlet port 44. The nutrition solution then exits the cylindrical body 34 of the holding means 28 through the channel 40 and is guided through the second channel 50 to the nutrition solution outlet port 46 where it is discharged. The nutrition solution can then e.g. be filtered from dead cells and/or circulating cells and recirculated back to the inlet port 44.

In addition, gas supply lines (not shown) are connected to the gas inlet and outlet ports 62, 64 such as to exchange gas with the nutrition solution flowing in the first and second channels 48, 50 via the corresponding gas exchange interfaces 52. By the gas exchange, the gas content of the nutrition solution, such as the CO₂ content can be maintained at physiological conditions that specific cells encounter in-vivo, e.g. a 5% content of CO₂.

At any time during cell seeding and/or conditioning on the scaffold patch 30, the whole bioreactor 10, while still connected to the respective supply lines (not shown) can be placed on a microscope table, and the current state of the tissue growing and/or conditioning on the scaffold 30 can be examined by ordinary microscopy or fluorescence microscopy. For this purpose, an image of the lower side of the scaffold 30 can be taken through the glass plate 68 and the opening 66 in the bottom portion 12, while the scaffold 30 is illuminated through the opening 70 and the glass plate 72. Since the gas and heat exchange means are provided within in the bioreactor 10, the physiological conditions can be maintained, while the scaffold 30 is under optical examination, and the tissue growth and/or conditioning can be maintained during or picked up immediately after the inspection. This means that an optical inspection of the tissue growth and/or conditioning can be made at any time or continuously during the tissue growth and/or conditioning process.

To further facilitate the optical inspection, the length and width of the bioreactor 10 are chosen to correspond to the length and width of a standard microtiter plate as routinely used in microscopy. In the present example, the length of the bioreactor 10 housing is 127.9 mm and the width is 85.6 mm.

While in conventional tissue engineering, tissues are only examined after the growth process is finished, the method and bioreactor of the invention allow obtaining valuable insights in the tissue growth and/or conditioning process as such, which promotes a better understanding thereof. This way formerly inaccessible information can be gained that will help to identify favorable combinations of scaffold types and cell types in the nutrition solution and to optimize the processing parameters, including the behavior of the colonized cells to flow shear stresses under physiological conditions. Also, by inspecting the tissue already during early stages of tissue growth and/or conditioning, clearly non-working experiments can be immediately terminated, such as to save time and resources.

Note that the bioreactor 10 of the preferred embodiment is devised for particularly small scaffold patches 30 having an area of less than 400 mm², typically even less than 200 mm² and preferably less than 100 mm². Accordingly, the number of cells needed to seed such scaffold patches is still moderate and can be cultivated in reasonable time. Still, the behavior of the tissue under physiological flow conditions can be directly discerned, since the flow of nutrition solution across the surface of the scaffold patch 30 can be easily, reliably and reproducibly controlled.

Finally, the construction of the bioreactor 10 shown in Figs. 1 to 5 is rather simple and can be manufactured at low cost. One of the reasons for the low cost is that the first and second nutrition solution channels 48, 50 and the gas channels 56, 58 are formed by bores in wall portions and that hence no additional components or complicated assembly are needed. Accordingly, the bioreactor 10 can be manufactured as a disposable article.

The embodiment described above and the accompanying figures merely serve to illustrate the method and bioreactor of the invention and should not be taken to indicate any limitation thereof. The scope of the patent is solely determined by the following claims.

### LIST OF REFERENCE SIGNS

- 10: bioreactor
- 12: bottom portion
- 14: top portion
- 16: cavity
- 16a: first compartment
- 16b: second compartment
- 18: partitioning wall
- 20: heating fluid inlet port
- 22: narrow opening
- 24: heating fluid outlet port
- 26: cylindrical structure
- 28: holding means
- 30: small scaffold patch
- 32: annular seat
- 34: cylindrical body
- 36: clamping ring
- 38: enlarged diameter portion
- 40: channel
- 42: recess
- 44: nutrition solution inlet port
- 46: nutrition solution outlet port
- 48: first channel
- 50: second channel
- 52: gas exchange interfaces
- 54: membrane
- 56: first gas channel
- 58: second gas channel
- 60: outlet
- 62: gas inlet port
- 64: gas outlet port
- 66: opening
- 68: light transparent glass plate
- 70: opening
- 72: light transparent glass plate
- 74: sealing ring
- 78: lid
- 80: threaded portion of the lid 78
- 82: threaded portion of the opening 70

## Claims

1. A method of examining tissue growth and/or conditioning and/or adhesion properties of cells on a scaffold (30), comprising the following steps:
- fixing a scaffold (30) to a holding means (28) disposed or disposable in a perfusion bioreactor (10),
- generating a flow of nutrition solution along the surface of said scaffold (30) held by said holding means (28),
- generating and/or maintaining physiological conditions in said nutrition solution flowing along said scaffold (30) using heat exchange means (20, 24, 48, 50) and/or gas exchange means (52, 54, 56, 58, 60, 62, 64) associated with said bioreactor (10), such as to allow growth and/or conditioning of cells on said scaffold (30), and
- optically inspecting, in particular by microscopy, the colonized scaffold (30) held by said holding means (28) through a window (66, 68, 70, 72) provided in said perfusion bioreactor (10) at different times or continuously during tissue growth and/or conditioning of cells on said scaffold (30) while maintaining said physiological conditions in said perfusion bioreactor (10).

2. The method of claim 1, wherein said flow generated along said scaffold (30) has a flow speed of 0.04 to 167 mm/s, preferably at least 33 mm/s, and/or wherein said scaffold (30) has a size of 400 mm² or less, preferably 200 mm² or less, and preferably even less than 100 mm².

3. The method of claim 1 or 2, wherein said bioreactor (10) is a bioreactor according to one of claims 4 to 15.

4. A perfusion bioreactor (10) for use in tissue engineering, comprising:
- a housing (12, 14),
- holding means (28) disposed or disposable in said housing (12,14), for holding a scaffold (30) to be seeded with cells,
- means (40, 48, 50) for directing a flow of nutrition solution along the surface of a scaffold (30) when held in said holding means (28), and
- at least one window, preferably two opposite windows (66, 68, 70, 72), said at least one window (66, 68, 70, 72) being transmissive for at least one of visible light, IR or UV radiation and arranged such as to allow optical, particularly microscopy inspection of the scaffold (30) when held in said holding means (28).

5. The perfusion bioreactor (10) of claim 4, further comprising heat exchange means (20, 24, 48, 50) and/or gas exchange means (52, 54, 56, 58,60, 62, 64) for use in generating and/or maintaining physiological conditions in said nutrition solution flowing along said scaffold (30).

6. The perfusion bioreactor (10) of claim 4 or 5, wherein said means (40, 48, 50) for directing said flow of nutrition solution is capable of allowing a flow of nutrition solution along said scaffold (30) having a flow speed of 0.04 to 167 mm/s, preferably at least 33 mm/s, and/or wherein said holding means (28) is dimensioned to hold scaffolds (30) having a size of 400 mm² or less, preferably 200 mm² or less, and most preferably 100 mm² or less.

7. The perfusion bioreactor (10) of one of claims 4 to 6, wherein said housing (12, 14) has an opening (70) for inserting the holding means (28) to and removing said holding means (28) from said housing (12, 14), and a lid (78) or cap for closing the opening (70).

8. The perfusion bioreactor (10) of one of claims 4 to 7, wherein said holding means (28) comprises clamping means for clamping the scaffold (30), and in particular an annular seat (32) for receiving said scaffold (30) and an annular clamping ring (36) that can be clamped in a position suitable for pressing said scaffold (30) against said seat (32), wherein the clamping ring is preferably dimensioned to allow a selective cell seeding of a scaffold area of 400 mm² or less, preferably 200 mm² or less and most preferably 100 mm² or less.

9. The perfusion bioreactor (10) of one of claims 4 to 8, wherein the holding means (28) is part of a preferably cylindrical substrate chamber through which said nutrition solution may be fed and that is closed by said at least one window (68, 72), and/or wherein said holding means (28) comprises a channel (40) for directing nutrition solution to flow along said surface of said scaffold (30).

10. The perfusion bioreactor (10) of one of claims 4 to 9, further comprising inlet and outlet ports (44, 46) for nutrition solution, a first channel (48) for guiding nutrition solution from said inlet port (44) to the holding means (28) and/or a second channel (50) for guiding nutrition solution from said holding means (28) to said outlet port (46).

11. The perfusion bioreactor (10) of one of claims 5 to 10, wherein said heat exchange means comprise inlet and outlet ports (20, 24) for feeding and discharging a heating fluid to and from said bioreactor (10), respectively,
wherein the heating fluid inlet port (20) is preferably located closer to the nutrition solution outlet port (46) and the heating fluid outlet port (24) is located closer to the nutrition solution inlet port (44).

12. The perfusion bioreactor. (10) of claim 11, wherein the housing comprises a cavity (16) for receiving said heating fluid, and at least a part of said first and/or second channel(s) (48, 50) is (are) disposed in said cavity (16) such as to be at least partially surrounded directly or indirectly by said heating fluid.

13. The perfusion bioreactor (10) of claim 12, wherein said heating fluid receiving cavity (16) is partitioned into at least two compartments (16a, 16b) by at least one partitioning wall (18),
said partitioning wall (18) having at least one opening (22) allowing a flow of heating fluid from one compartment (16a, 16b) to another,
wherein said first and/or second nutrition solution channel(s) (48, 50) is (are) preferably formed by a bore in said partitioning wall (18).

14. The perfusion bioreactor (10) of one of claims 5 to 13, wherein said gas exchange means comprise one or more gas exchange interface(s) (52) for exchanging gas with said nutrition solution,
wherein said one or more gas exchange interface(s) (52) is (are) preferably associated with said first and/or second nutrition solution channels (48, 50),
wherein said one or more gas exchange interface(s) (52) preferably comprise(s) a gas-permeable membrane (54) and/or is (are) connected with a gas inlet or outlet port (62, 64) via a gas channel (56, 58),
wherein said gas channel (56, 58) is preferably formed by a bore in a housing wall (14).

15. The perfusion bioreactor (10) of one of claims 4 to 14, wherein the length and width of the housing (12, 14) correspond to the length and width of a standard microtiter plate, wherein in particular the length is 127.9 mm +/- 5% and the width is 85.6 mm +/- 5%.
